Europäisches Patentamt

European Patent Office

Office européen des brevets

(11) Veröffentlichungsnummer : **0 478 985 B1**

# (12) EUROPÄISCHE PATENTSCHRIFT

(45) Veröffentlichungstag der Patentschrift :
**02.03.94 Patentblatt 94/09**

(51) Int. Cl.$^5$ : **F02D 19/08,** G01N 33/28

(21) Anmeldenummer : **91115143.9**

(22) Anmeldetag : **07.09.91**

(54) Verfahren und Einrichtung zur Nutzung von Brennstoffen mit Alkoholzusätzen für eine Brennkraftmaschine.

(30) Priorität : **01.10.90 DE 4031008**

(43) Veröffentlichungstag der Anmeldung :
**08.04.92 Patentblatt 92/15**

(45) Bekanntmachung des Hinweises auf die
Patenterteilung :
**02.03.94 Patentblatt 94/09**

(84) Benannte Vertragsstaaten :
**DE FR GB IT**

(56) Entgegenhaltungen :
**DE-A- 2 259 323
GB-A- 2 136 118**

(73) Patentinhaber : **PIERBURG GMBH
Alfred-Pierburg-Strasse 1
D-41460 Neuss (DE)**

(72) Erfinder : **Härtel, Günter
Am Vogelbusch 16
W-4040 Neuss 21 (DE)**
Erfinder : **Schürfeld, Armin
Wagnerplatz 13
W-4005 Meerbusch 2 (DE)**
Erfinder : **Lösing, Karl-Heinrich, Dr.
Heidestrasse 88a
W-4234 Alpen (DE)**
Erfinder : **Thönnessen, Dieter
Ackerstrasse 29
W-4060 Viersen 11 (DE)**
Erfinder : **Remde, Ulrich
Leipziger Strasse 18
W-4005 Meerbusch 3 (DE)**

EP 0 478 985 B1

**Beschreibung**

Die Erfindung betrifft ein Verfahren und Einrichtung zur Nutzung von Brennstoffen mit Alkoholzusätzen für eine Brennkraftmaschine.

Neben Superbenzin, Normalbenzin und Dieselkraftstoff sind auch Alkohole wie Methanol, Ethanol, Propanol, Isobutanol u. a. als Brennstoffe für Verbrennungsmaschinen geeignet.

Für die Leistung des Motors ist der Heizwert des brennfähigen Brennstoff-Luft-Gemisches maßgebend. Bei stöchiometrischem Mischungsverhältnis liegt der Gemischheizwert für alle praktisch verwendbaren flüssigen Brennstoffe und Flüssiggase zwischen 700 und 735 kcal/kg; sie sind also fast gleich und Leistungseinbußen des Motors durch Zumischen von Methanol u. a., sind nicht zu befürchten. Auch die meisten sonstigen Eigenschaften der Alkohole sind so, daß sie sich ohne weiteres mit üblichen Flüssigbrennstoffen mischen lassen bzw. keine Störungen beim praktischen Motorbetrieb hervorrufen.

Wünschenswert ist eine Alkoholzusammensetzung vor allem aus folgenden Gründen: Alkohole eignen sich als Veredelungsbrennstoff für minderwertige Benzine. Durch Zugabe von z. B. Methanol zu Normalbenzin kann die Klopffestigkeit wesentlich erhöht werden, so daß mit diesem Gemisch auch höher verdichtete Verbrennungsmotoren gefahren werden können. Es werden umweltschädliche Antiklopfmittel entbehrlich.
Hinzu kommt, daß Alkohole zunehmend in den USA aus Umweltschutzgründen an Bedeutung gewinnen, insbesondere durch verschärfte Vorschriften in Kalifornien.

Im übrigen lassen sich Alkohole, insbesondere Methanol, auf relativ billige Weise aus Kohle herstellen und sie stehen daher auch in ferner Zukunft in großen Mengen zur Verfügung. Besonders Ethanol ist umweltfreundlich, wenn es aus pflanzlichen Produkten gewonnen wird (biologischer Kreislauf).
Alkohole sind daher ein geeignetes Streckmittel für die nur noch begrenzt verfügbaren Erdölbrennstoffe.

Nachteilig ist jedoch, daß der spezifische Mindestluftbedarf für eine vollständige Verbrennung bei Alkoholen geringer ist als bei herkömmlichen Brennstoffen. Bei derselben angesaugten Luftmenge muß dem Motor bei Alkohol- oder Benzin/Alkohol-Mischbetrieb also eine entsprechend höhere Brennstoffmenge zugeführt werden, um ein stöchiometrisches Luft-Brennstoff-Verhältnis zu erreichen. Dies macht eine entsprechende Justierung der Gemischaufbereitungseinrichtung des Verbrennungsmotors erforderlich. Da jedoch gegebenenfalls Alkohole in schwankenden Anteilen zugemischt werden oder zumindest sich beim Nachtanken unterschiedlicher Brennstoffsorten im Kraftfahrzeugtank ergeben, muß entsprechend dem Alkoholanteil im Brennstofftank das erforderliche Luft-Brennstoff-Verhältnis für stöchiometrisches Gemisch im elektronischen Steuergerät für den Gemischbildner neu bestimmt werden.

In der DE-OS 40 19 188 ist eine Mehrstoffmaschinensteuerung mit einem Kraftstoffsensor zur Erfassung des Mischungsanteils von Methanol eines Brennstoff-Methanolgemisches offenbart, dessen Ausgangssignal zum Nachstellen eines gespeicherten Maschinentreibstoff-Steuerungsparameters benutzt wird. Ein bevorzugter Kraftstoffsensor ist ein kapazitiver dielektrischer Sensor, der zwei im Abstand voneinander angeordnete Elektrodenplatten enthält, die in den Brennstoff eingetaucht sind. Die Dielektrizitäts konstante des Brennstoffes wird durch Messung der Kapazität zwischen den beiden Elektrodenplatten erfaßt, und zwar der zugemischte Anteil an Methanol wird aus der Dielektrizitätskonstanten abgeleitet. Dieses Meßprinzip zeigt jedoch eine starke Anfälligkeit bei Dampfblasenbildung. Desweiteren ist dieser Kraftstoffsensor nur in der Lage, den Zumischanteil eines vorher bekannten Alkohols (z. B. Methanol) zu erfassen.

Aus der DE-OS 22 59 323 ist eine Vorrichtung zur automatischen Anpassung einer Brennkraftmaschine an die Anforderungen der wahlweisen Verwendung verschiedenartiger flüssiger Brennstoffe bekannt, die einen brennstoffdurchflossenen, bis auf die Zu- und Ablauföffnungen für den Kraftstoff geschlossenen Meßbehälter aufweist, in dem eine Teilmenge des Brennstoffes unter dem Einfluß zugeführter Wärme verdampft wird, wobei die relative Größe des dampfgefüllten Raumes im Inneren des Meßbehälters als Maß zur Anpassung an die verschiedenen Brennstoffarten dient.

Nachteilig hierbei ist, daß nur festgestellt werden kann, ob ein bestimmter Anteil über- oder unterschritten wird, wobei auch hier dieser Anteil vorher bekannt sein muß, um den Meßfühler dieser Vorrichtung in einer bestimmten Höhe in dem Meßbehälter anzuordnen.

Hiervon ausgehend liegt der Erfindung die Aufgabe zugrunde, ein Verfahren und eine Einrichtung zur Nutzung von Brennstoffen mit Alkoholzusätzen für eine Brennkraftmaschine eingangs genannter Art derart zu gestalten, daß o.g. Nachteile vermieden werden.

Diese Aufgabe wird durch die Merkmale der Ansprüche 1 bis 7 hinsichtlich des Verfahrens gelöst.
Die Ansprüche 8 bis 15 beziehen sich auf eine Einrichtung, die nach den Verfahren betrieben wird und vorteilhafte Weiterbildungen dieser Einrichtung.
Die Erfindung macht sich die Tatsache zunutze, daß sich bei Mischungen von handelsüblichen Brennstoffen (Benzin, Diesel) mit Alkohol (Methanol, Ethanol u.s.w.) beim Beheizen einer Probe mit definierter Leistung im Bereich der Siedepunkte von Methanol bzw. Ethanol aufgrund der Verdampfungswärme der beigemischten Alkoholsorten und -mengen eine mehr oder weniger stark ausgeprägte Verzögerung im Temperaturanstieg zeigt. Dabei ist das Ausmaß der Verzögerung des Temperaturanstieges abhängig von der prozentualen Beimengung des Alkohols und die Lage des Temperaturbereichs, in dem sich die Verzögerung des Temperaturanstieges vollzieht, abhängig von der Art der Alkoholbeimengung.

Um diese erfindungsgemäßen Verfahren durchzuführen, wird eine erfindungsgemäße Einrichtung vorgeschlagen, die den Temperaturbereich erfaßt, in dem sich die Temperaturanstiegsverzögerung vollzieht, wodurch die Alkoholsorte definiert ist, und die das Ausmaß der Verzögerung des Temperaturanstieges erfaßt, wodurch die prozentuale Beimengung dieses Alkohols definiert ist. Die während der Verdampfung gewonnenen Meßwerte (Temperaturlage und-/oder Zeit) und/oder ein in einem Steuergerät aufbereitetes Korrektursignal bewirken eine Änderung der Brennstoffzufuhr bzw. der Luftzufuhr der Brennkraftmaschine.

Mit dieser Erfindung wird es möglich, verschiedene Alkohole und deren prozentuale Anteile im Brennstoff zu unterscheiden, in dem z. B. nach jedem Motorstart eine Messung durchgeführt wird, nach deren Ergebnis eine Beeinflussung der Brennkraftmaschine differenziert nach den beigemischten Alkoholsorten erfolgen kann.

Vorteilhafterweise bietet der lineare Zusammenhang der Verzögerungszeiten in Relation zum Alkoholgehalt und die weitgehende Unabhängigkeit vom Siedeverlauf des Ottobrennstoffes durch die Nutzung der Verdampfungswärme der Alkohole eine gute Voraussetzung für eine hohe Meßgenauigkeit.
Eventuell im Brennstoff vorhandene Wasseranteile sind aufgrund deren geringen spezifischen Wärme im Vergleich zur Verdampfungswärme der Alkohole von vernachlässigbar geringem Einfluß.
Ein weiterer Vorteil ist die Dampfblasenunempfindlichkeit.

Anhand der Figuren 1 bis 7 wird die Erfindung näher erläutert. Es zeigt:

**Fig. 1** einen Schnitt durch eine Verdampfungseinrichtung,

**Fig. 2** den zeitlichen Temperaturverlauf eines Brennstoff-Methanol-Gemisches,

**Fig. 3** einen Schnitt durch ein PTC-Heizelement,

**Fig. 4** einen Schnitt durch eine andere Ausführungsform eines PTC-Heizelementes,

**Fig. 5** einen Schnitt durch eine weitere Ausführungsform eines PTC-Heizelementes,

**Fig. 6** einen Schnitt durch eine andere Ausführungsform einer Verdampfungseinrichtung.

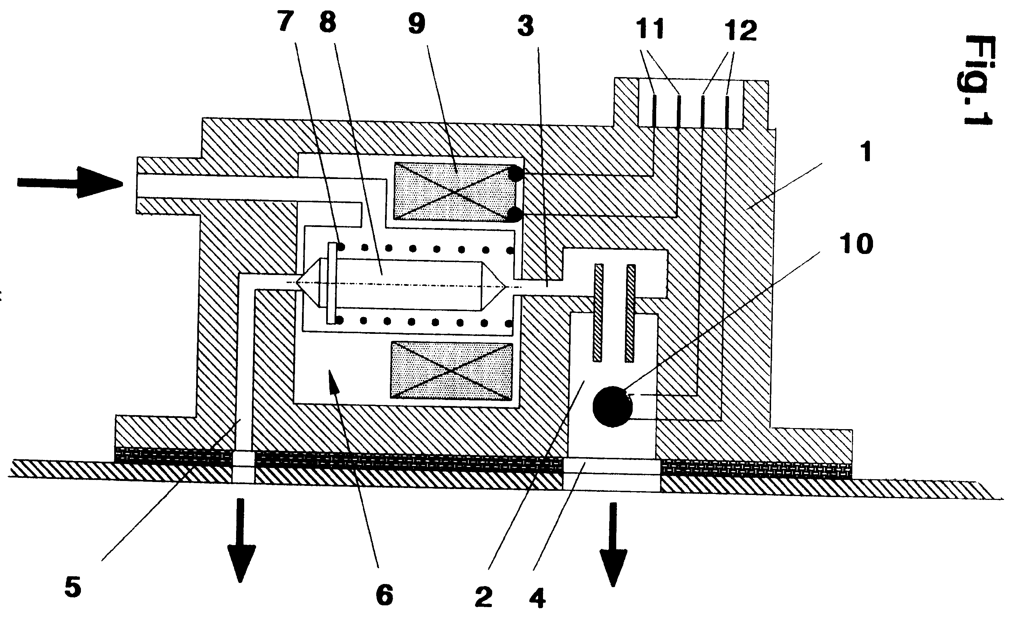

Fig. 1 zeigt eine Einrichtung zur Verdampfung einer Brennstoffprobenmenge bestehend aus einem Gehäuse 1 mit einer Verdampfungskammer 2, die einen Zulauf- und Ablaufkanal 3, 4 aufweist, wobei der Zulaufkanal 3 und ein Rücklaufkanal 5 von einer Ventileinheit 6 geöffnet bzw. geschlossen wird, deren gegen die Kraft einer Feder 7 wirkender Schließkörper 8 von einer Magnetspule 9 betätigt wird. Die Magnetspule 9 und ein in der Verdampfungskammer 2 angeordnetes, hier als PTC ausgeführtes Heizelement 10, das bei dieser Ausführungsform auch als Temperaturmeßstelle und Probennahme dient, werden über Steuerleitungen 11, 12 von einem nicht dargestellten Steuergerät angesteuert.

Funktionsweise unter Anwendung des Verfahrens nach Anspruch 1

Befindet sich die Einrichtung zur Verdampfung einer Brennstoffprobenmenge in Ruhestellung, verschließt der Schließkörper 8 durch die Kraft der Feder 7 den Rücklaufkanal 5 und Brennstoff gelangt kontinuierlich über den geöffneten Zulaufkanal 3 in den Verdampfungsraum 2, benetzt das Heizelement 10 und strömt über den Ablaufkanal 4 zurück in den Brennstofftank.

Bei Beginn eines Meßzyklus verschließt der Schließkörper 8 mit Hilfe der über Steuerleitungen 11 von dem Steuergerät angesteuerten Magnetspule 9 den Zulaufkanal 3 und öffnet den Rücklaufkanal 5, so daß kein weiterer Brennstoff in die Verdampfungskammer 2 gelangt, die sich über den Ablaufkanal 4 so weit entleert, bis nur noch das Heizelement 10 benetzt ist.

Nach Verfahrensschritt eins wird durch Anlegen einer Spannung am Heizelement 10 die Brennstoffprobenmenge aufgeheizt, was zum Abdampfen des Alkoholanteils im Brennstoff führt.

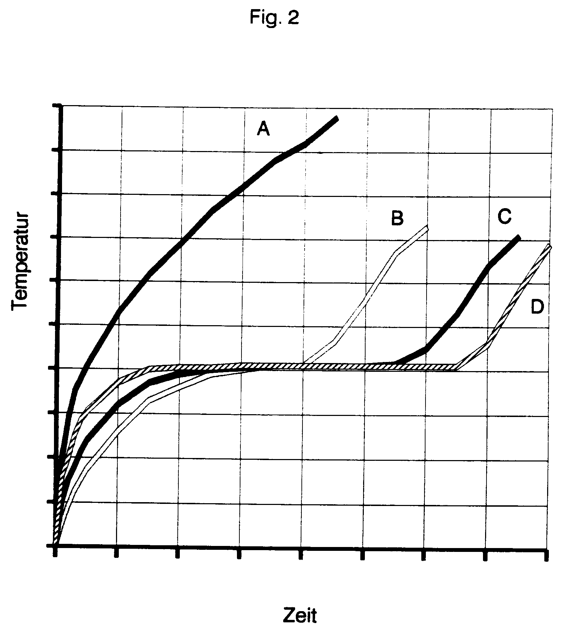

Fig. 2 zeigt beispielhaft den zeitlichen Temperaturverlauf (T=f(t)) eines Brennstoff-Methanol-Gemisches, wobei die Kurve A einem 0%-Anteil Methanol (normaler Brennstoff) und die Kurven B-D einen 50-100%-Anteil Methanol entsprechen. Es ist deutlich eine Verzögerung des Temperaturanstieges zu erkennen, die in etwa auf dem Temperaturniveau der Siedetemperatur von Methanol liegt (ca. 65°C).

Nach Verfahrensschritt zwei wird fortlaufend die Brennstoffprobentemperatur gemessen - in diesem Anwendungsfall indirekt über den PTC 10 durch Messung der Stromstärke, da sich mit dessen Erwärmung der Widerstand verändert, wodurch sich bei konstanter Spannung der Stromfluß reziprok verändert - und der Temperaturgradient der Brennstoffprobenmenge (Differentialquotient $\frac{dT}{dt}$ der Aufheizkurve T = f (t)) ermittelt.

In dem dritten Verfahrensschritt wird bei einer festgelegten Abweichung, d. h. einer bestimmten prozentualen Abweichung des ermittelten Temperaturgradienten von einem vorgegebenen Temperaturgradienten eines üblichen Brennstoffes die Brennstoffprobentemperatur gemessen und eine Verdampfungszeitmessung gestartet, wobei die Heizleistung ab diesem Verdampfungszeitmessungsstart konstant gehalten wird. Bis zum Start der Verdampfungszeitmessung kann die Brennstoffprobenmenge mit variabler oder konstanter Spannung aufgeheizt werden.

In dem vierten Verfahrensschritt wird bei Feststellung eines mit dem Ausgangstemperaturgradienten des üblichen Brennstoffs vergleichbaren Temperaturgradienten die Probentemperatur gemessen, die Verdampfungszeitmessung gestoppt und die gesamte Verdampfungszeit festgestellt.

Verfahrensschritt drei und vier werden anhand der Fig. 2 nochmals erläutert. Liegt z. B. eine 50%ige Alkoholbeimischung vor (Kurve B), dann wird die Zeitmessung gestartet, wenn die Kurve B aus ihrem ersten steilen Anstieg in einen flacheren Verlauf übergeht (Detektion der Alkoholsorte durch das Temperaturniveau) und wenn nach einer bestimmten Verdampfungszeit die Kurve B wieder in einen steilen Verlauf übergeht, wird die Zeitmessung gestoppt, wobei die Gesamtzeit zwischen Start und Ende der Zeitmessung eine Aussage über den prozentualen Anteil der Alkoholsorte ermöglicht.

In dem fünften Verfahrensschritt wird aus den ermittelten Temperatur- und Verdampfungszeitwerten durch einen Vergleich mit in einem Kennfeldspeicher des Steuergerätes abgespeicherten Vergleichswerten oder alkoholsortenspezifischen Kennfeldern ein Korrektursignal gebildet, das in dem sechsten Verfahrensschritt, falls erforderlich, zur Änderung von Motorsteuerungsgrößen z. B. der Brennstoff- bzw. Luftzuführung und so weiter verwendet werden kann.

Das gebildete Korrektursignal wird in einem siebten Verfahrensschritt gespeichert, bis es bei erneuter Brennstoffprobenmessung bestätigt bzw. aktualisiert wird.

Nach dem fünften Verfahrensschritt ist der Meßzyklus der Verdampfungseinrichtung beendet, die Stromversorgung zur Magnetspule 9 und zum Heizelement 10 wird unterbrochen, der Schließkörper 8 wird durch die Federkraft der Feder 7 in seine Ruhestellung gebracht, wobei der Rücklaufkanal 5 gesperrt und der Zulaufkanal 3 geöffnet ist und die Verdampfungskammer von frischem Brennstoff durchströmt, gespült und gekühlt wird.

Das Verfahren nach Anspruch 2 läuft nach dem ersten Verfahrensschritt gegenüber dem des Anspruchs 1 in einer abgeänderten Form ab, wobei im zweiten Verfahrenschritt der Temperaturanstieg der Brennstoffprobenmenge überwacht wird, bis eine erste Temperaturschwelle unterhalb des Siedebereiches eines Alkohols erreicht,

im dritten Verfahrensschritt bei Überschreiten der ersten Temperaturschwelle die Verdampfungszeitmessung gestartet wird, wobei von einem reproduzierbaren Heizleistungsverlauf ausgegangen wird, und

im vierten Verfahrensschritt der Temperaturanstieg überwacht wird bis eine zweite Temperaturschwelle oberhalb des Siedebereiches eines Alkohols erreicht wird, und dann die Verdampfungszeitmessung gestoppt wird und die gesamte Verdampfungszeit festgestellt wird. Der fünfte, sechste und siebte Verfahrensschritt läuft dann wie bereits oben beschrieben ab.

Es ist auch denkbar, zusätzlich eine weitere Temperaturschwelle vorzusehen, wobei dann z. B. eine erste Temperaturschwelle unterhalb des Siedebereiches von Methanol, eine zweite Temperaturschwelle zwischen den Siedebereichen von Methanol und Ethanol und eine dritte Temperaturschwelle oberhalb des Siedebereiches von Ethanol liegt.

Bei Überschreitung der ersten Temperaturschwelle wird eine erste Verdampfungszeitmessung gestartet, die bei Überschreiten der zweiten Temperaturschwelle gestoppt wird (dann Detektion Methanol durch das Temperaturniveau und Feststellung des prozentualen Anteils von Methanol aufgrund der Länge der Verdampfungszeit möglich), oder die nach Überschreitung der zweiten Temperaturschwelle nach einer verhältnismäßig kurzen Zeitdauer erneut gestartet wird und bei Überschreitung der dritten Temperaturschwelle gestoppt wird (dann Detektion Ethanol durch das höhere Temperaturniveau und Feststellung des prozentualen Anteils von Ethanol aufgrund der Länge der Verdampfungszeit möglich) oder die nach Überschreitung der dritten Temperaturschwelle nach einer verhältnismäßig kurzen Zeitdauer gestoppt wird (Detektion normaler Brennstoff aufgrund des Temperaturniveaus).

Bei der nach Fig. 1 beschriebenen Einrichtung kann die Temperaturmessung auch von einem separaten Temperatursensor durchgeführt werden, der dann möglichst nahe am Heizelement angeordnet ist bzw. mit diesem in Körperkontakt steht, so daß ein guter Wärmefluß vorhanden ist. Außerdem kann die Ventileinheit so angeordnet und ausgeführt sein, daß z. B. nur der Zulaufkanal zur Verdampfungskammer je nach Bedarf geöffnet bzw. geschlossen wird.

Die sich immer weiter verschärfenden Abgasvorschriften machen es erforderlich, daß auch kleine und geringste Arbeitszeiträume einer Brennkraftmaschine (z. B. Kaltstart, Warmlaufphase, Heißstart und so weiter) immer mehr einer Optimierung der Abgasemissionen unterzogen werden müssen. Aus diesem Grund wird das bisher beschriebene Verfahren wie folgt weiter verbessert.

Bei einem Start (Kalt-Heißstart) der Brennkraftmaschine wird sofort ein Meßzyklus zur Korrektursignalbildung ausgelöst, ein neues Korrektursignal steht jedoch erst nach einer gewissen Zeit zur Verfügung, die benötigt wird, um die Verfahrensschritte eins bis sieben zu durchlaufen.

Für den Start und bis zur Vorlage eines aktuellen Korrektursignals wird das letzte gespeicherte Korrektursignal verwendet.

Nach dem Start werden generell mehrere Messungen in Folge durchgeführt, bis sich der Meßwert stabilisiert hat.

Damit wird erreicht, daß nach einem Tankstop die mo-

mentan den Einspritzventilen zugeführte Brennstoff-mischung erfaßt wird, wenn die Meßeinrichtung vorzugsweise in der Rücklaufleitung zum Tank angeordnet wird.

Es ist jedoch auch denkbar, das letzte gespeicherte Korrektursignal in Abhängigkeit des Füllstandes des Brennstofftanks zu korrigieren bzw. solange zu übersteuern, bis ein neues Korrektursignal der Verdampfungseinrichtung vorliegt.

Hierbei wird dann zusätzlich zum gespeicherten Korrektursignal beim Abstellen der Brennkraftmaschine der Füllstand des Brennstofftanks erfaßt und gespeichert, und beim Start mit dem Istbefüllungszustand verglichen, wobei noch unterschieden werden kann, wie schnell der Istbefüllungszustand erfaßt wird. Liegt ein Istbefüllungszustand erst nach dem Vorliegen des aktuellen Korrektursignals vor (langsame Tankanzeige), wird folgender Verfahrensablauf A vorgeschlagen, bei einer schnellen Tankanzeige, also bei Vorliegen des Istbefüllungszustandes vor Vorlage des aktuellen Korrektursignals wird ein Verfahrensablauf B vorgegeben.

Verfahrensablauf A (langsame Tankanzeige)

Der Start erfolgt mit Verwendung des letzten gespeicherten Korrektursignals, gleichzeitig wird der beim Abstellen der Brennkraftmaschine gespeicherte Tankbefüllungszustand bewertet, d. h. es wird festgestellt, ob der Brennstofftank, z. B. $\geqq$ halb voll oder $\leqq$ halb voll war.

Bei Feststellung $\geqq$ halb voll wird bis zur Vorlage des aktuellen Korrektursignals das gespeicherte Korrektursignal verwendet, da angenommen wird, daß keine Betankung nach dem Abstellen der Brennkraftmaschine erfolgte, bzw. falls eine Betankung doch erfolgte war, eine Verfälschung durch die zugetankte Menge nicht gravierend erscheint.

Bei Feststelllung $\leqq$ halb voll wird angenommen, daß eine Betankung erfolgt war und das letzte gespeicherte Korrektursignal wird auf ein mittleres Korrektursignal (z. B. das eines normalen Brennstoffes) nach einer Vorgabefunktion (linear, e-Funktion, treppenförmig u.s.w.) korrigiert.

Nach Vorlage eines aktuellen Korrektursignals der Verdampfungseinrichtung wird sofort mit diesem weitergefahren.

Verfahrensablauf B (schnelle Tankanzeige)

Bei Feststellung der Nichtänderung des Tankbefüllungszustandes wird bis zur Vorlage des aktuellen Korrektursignals das gespeicherte Korrektursignal verwendet.

Bei Feststellung der Änderung des Tankbefüllungszustandes läuft das Verfahren B so weiter wie das Verfahren A im Zustand der Feststellung $\leqq$ halb voll.

Im Betriebsfall einer $\lambda$ = 1-Regelung wird mit stöchiometrischem Gemisch gefahren. In dieser Betriebsphase ist es möglich, das Korrektursignal quantitativ zu überprüfen und im Falle einer Abweichung adaptiv anzugleichen.

Um die am Heizelement 10 haftende Brennstoffmenge den Erfordernissen bezüglich der Meßzeit und/oder der Reproduzierbarkeit der Probenmenge anzupassen, können eine Reihe von Modifikationen der Oberflächenbeschaffenheit und der Kontur des Heizelementes 10 von Vorteil sein.

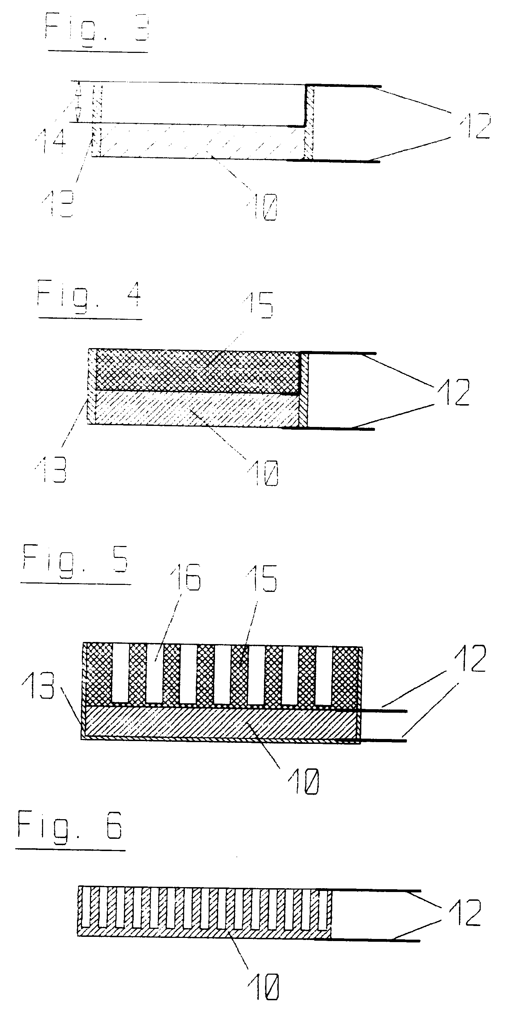

Fig. 3 zeigt ein als PTC-Element ausgebildetes Heizelement 10, das an seinem Umfang mit einem Mantel 13, vorzugsweise aus Material mit geringer Wärmekapazität und -leitung, umgeben ist, der die Oberseite des PTC-Elementes 10 um eine die Brennstoffprobenmenge definierende Kragenhöhe 14 überragt.

In einer weiteren Ausgestaltung des PTC-Elementes nach Fig. 4 ist der von der Kragenhöhe 14 gebildete Raum bündig mit einem Körper 15, vorzugsweise aus porösem Material (z. B. Sintermetall) aufgefüllt, wodurch eine gewisse Lageunempfindlichkeit der Ausführung nach Fig. 3 erreicht wird.

Es ist auch denkbar, das PTC-Element beidseitig mit einem Körper 15, z. B. aus Sintermetall, zu versehen und in beliebiger geometrischer Form auszubilden, wobei die Verbindung der porösen Schichten (Sintermetall) mit dem PTC-Element durch Kleben oder Löten erfolgen kann.

Zusätzlich kann der Körper 15 Ausnehmungen 16 aufweisen, wie die Fig. 5 zeigt, die in Form von Löchern, Schlitzen, Mehrecken oder sonstigen geometrischen Formen ausgebildet sein können. Hierbei ist das PTC-Element 10 vollständig abgekapselt, falls sich der direkte Kontakt des PTC-Elementes mit dem Kraftstoff als Nachteil erweisen sollte. Die Abdichtung gegenüber dem Mantel 13 und des Körpers 15 wird durch Löten oder Kleben mit einem brennstoffesten Kleber erreicht. Die Verwendung des hier als Lochplatte ausgeführten Körpers 15 ermöglicht einen schnelleren Kraftstoffaustausch, wenn sich dieser im porösem Material hinsichtlich der Kapillarwirkung zwischen den Meßvorgängen als problematisch herausstellen sollte.

Im Falle einer guten Verträglichkeit des Heizelement-Materials mit dem Brennstoff kann das Heizelement 10 als Lochplatte (wie in Fig. 6 dargestellt) ausgeführt sein, dessen Ausnehmungen 16 als Löcher ausgeführt sind, aber auch als Schlitze, Mehrecke u.s.w. dargestellt sein können, wobei die Ausnehmungen von deren Oberseite ausgehend durchgehend offen oder zur Unterseite geschlossen sind.

Sofern darstellbar, kann das als Lochplatte ausgeführte PTC-Element 10 auch aus porösem Material hergestellt sein.

Eine andere Ausführungsform einer Einrichtung zur Verdampfung einer Brennstoffprobenmenge (zeichnerisch nicht dargestellt) ist vorzugsweise im Gasvolumen eines Brennstofftanks eingebaut und besteht aus einem mit einem Zulauf- und Ablaufkanal versehenen Gehäuse, in dem ein PTC-Element 10 angeordnet und über Steuerleitungen 12 angesteuert wird. Der Ablaufkanal wird über ein 2/3-Wegeventil geöffnet und geschlossen. In den Meßpausen wird über eine entsprechende Schaltstellung des 2/3-Wegeventils das Gehäuse mit dem PTC-Element ständig mit Kraftstoff, z. B. aus dem Rücklauf des Gemischbildners zum Tank, durchflutet. Bei Meßbeginn wird das 2/3-Wegeventil durch Ansteuerung von einem nicht dargestellten elektronischen Steuergerät umgeschaltet, wodurch ein Rücklauf unter Umgehung des Gehäuses mit dem PTC-Element direkt in den Tank fließt. Gleichzeitig wird die Spannungsversorgung des PTC-Elementes eingeschaltet, die im Gehäuse befindliche Kraftstoffprobe beheizt und das Korrektursignal nach dem beschriebenen Verfahren gebildet. Nach Abschalten der Beheizung wird das 2/3-Wegeventil auf 'Fluten' des Gehäuses umgeschaltet. Nach dem 'Ausspülen' der erwärmten Restkraftstoffprobe aus dem Gehäuse besteht wieder Meßbereitschaft.

**Patentansprüche**

1. Verfahren zur Nutzung von Brennstoffen mit Alkoholzusätzen für eine mit einem elektronischen Gemischbildungssystem betriebene Brennkraftmaschine, bei dem mittels eines Kraftstoffsensors zur Erfassung des Alkoholanteils in einem Benzin-Brenngemisch ein Korrektursignal zur Änderung der Gemischzusammensetzung in einem Steuergerät erzeugt wird, wobei der Kraftstoffsensor aus einer Einrichtung zur Verdampfung einer Brennstoffprobenmenge besteht, **dadurch gekennzeichnet**, daß das Korrektursignal dadurch ermittelt wird, daß in einem

   - ersten Verfahrensschritt eine Brennstoffprobenmenge aufgeheizt wird, daß während der Aufheizung in einem

   - zweiten Verfahrensschritt die Brennstofftemperatur gemessen wird und fortlaufend der Temperaturgradient der Brennstoffprobenmenge (Differentialquotient $\frac{dT}{dt}$ der Aufheizkurve $T = f(t)$) ermittelt wird, daß in einem

   - dritten Verfahrensschritt bei einer festgelegten Abweichung des ermittelten Temperaturgradienten von einem vorgegebenen Temperaturgradienten eines üblichen Brennstoffes die Probenmengentemperatur gemessen wird und eine Verdampfungszeitmessung gestartet wird, wobei von einem reproduzierbaren Heizleistungsverlauf ausgegangen wird, daß in einem

   - vierten Verfahrensschritt bei Feststellung eines mit dem Ausgangs-Temperaturgradienten des üblichen Brennstoffes vergleichbaren Temperaturgradienten die Probetemperatur gemessen wird, die Verdampfungszeitmessung gestoppt und die gesamte Verdampfungszeit festgestellt wird, daß in einem

   - fünften Verfahrensschritt aus den ermittelten Temperatur- und Verdampfungszeitwerten durch einen Vergleich mit in einem Kennfeldspeicher des Steuergerätes abgespeicherten Vergleichswerten oder alkoholsortenspezifischen Kennfeldern ein Korrektursignal gebildet wird, daß in einem

   - sechsten Verfahrensschritt mittels des Korrektursignales Motorsteuerungsgrößen änderbar sind und daß in einem

   - siebten Verfahrensschritt das gebildete Korrektursignal gespeichert wird, bis es bei erneuter Brennstoffprobenmessung bestätigt bzw. aktualisiert wird.

2. Verfahren nach dem Oberbegriff des Anspruchs 1, **dadurch gekennzeichnet,** daß das Korrektursignal dadurch ermittelt wird, daß in einem

   - ersten Verfahrensschritt eine Brennstoffprobenmenge aufgeheizt wird, daß während der Aufheizung in einem

   - zweiten Verfahrensschritt die Brennstofftemperatur gemessen wird und der Temperaturanstieg überwacht wird, bis eine erste Temperaturschwelle unterhalb des Siedebereiches eines Alkohols erreicht wird, daß in einem

   - dritten Verfahrensschritt bei Überschreiten der ersten Temperaturschwelle eine Verdampfungszeitmessung gestartet wird, wobei von einem reproduzierbaren Heizleistungsverlauf ausgegangen wird, daß in einem

   - vierten Verfahrensschritt der Temperaturanstieg überwacht wird, bis eine zweite Temperaturschwelle oberhalb des Siedebereiches eines Alkohols erreicht wird, die Verdampfungszeitmessung gestoppt und die gesamte Verdampfungszeit festgestellt wird, daß in einem

   - fünften Verfahrensschritt aus den ermittelten Verdampfungszeitwerten durch einen Vergleich mit in einem Kennfeldspeicher des Steuergerätes abgespeicherten Vergleichswerten oder alkoholsortenspezifischen Kennfeldern ein Korrektursignal gebildet wird, daß in einem

   - sechsten Verfahrensschritt mittels des Korrektursignales Motorsteuerungsgrößen änderbar sind und daß in einem
- siebten Verfahrensschritt das gebildete Korrektursignal gespeichert wird, bis es bei erneuter Brennstoffprobenmessung bestätigt bzw. aktualisiert wird.

3. Verfahren nach Anspruch 2, **dadurch gekennzeichnet**, daß zusätzlich eine oder mehrere Temperaturschwellen vorgesehen werden, bei deren Überschreitung innerhalb einer festgelegten Zeitspanne nach dem ersten Start der Verdampfungszeitmessung eine neue Verdampfungszeitmessung gestartet wird.

4. Verfahren nach Anspruch 1, 2 oder 3, **dadurch gekennzeichnet**, daß die Brennstoffprobenmengen bis zum Start der Verdampfungszeitmessung mit konstanter oder variabler Spannung aufgeheizt wird.

5. Verfahren nach Anspruch 1, 2, 3 oder 4, **dadurch gekennzeichnet**, daß das im siebten Verfahrensschritt gespeicherte Korrektursignal auch bei einem Wiederstart der Brennkraftmaschine verwendet wird, bis es nach erneuter Brennstoffprobenmessung bestätigt bzw. aktualisiert wird.

6. Verfahren nach Anspruch 1, 2, 3 oder 4, **dadurch gekennzeichnet**, daß bei einem Start der Brennkraftmaschine ab einem bestimmten Füllstand des Brennstofftanks beim Abstellen der Brennkraftmaschine bis zur Vorlage des aktuellen Korrektursignals kein Korrektursignal oder ein Korrektursignal, das dem des normalen Brennstoffes entspricht, vorgegeben wird.

7. Verfahren nach Anspruch 5 oder 6, **dadurch gekennzeichnet**, daß bei Vorliegen eines Lambda-Signals eine Adaption des Korrektursignal erfolgen kann.

8. Einrichtung mit Mitteln zur Durchführung des Verfahrens nach den Ansprüchen 1-7, wobei die Einrichtung zur Verdampfung einer Brennstoffprobenmenge aus einem Gehäuse besteht mit einer mittels eines Heizelementes beheizbaren, Sensoren aufweisenden Verdampfungskammer mit Zu- und Ablaufkanälen, **dadurch gekennzeichnet**, daß die Befüllung der Verdampfungskammer (2) über eine Ventileinheit (6) gesteuert wird, die einen mit einer Feder (7) zusammenwirkenden Ventilschließkörper (8) aufweist, der mit Hilfe einer Magnetspule (9) die Zu- und Abflußkanäle (3, 4) öffnet und schließt.

9. Einrichtung nach Anspruch 8, **dadurch gekennzeichnet**, daß das Heizelement ein PTC-Element (10) ist.

10. Einrichtung nach Anspruch 8 oder 9, **dadurch gekennzeichnet**, daß in der Verdampfungskammer (2) ein Temperatursensor in der Nähe bzw. in Kontakt mit dem Heizelement (10) der Verdampfungskammer (2) angeordnet ist.

11. Einrichtung nach Anspruch 8 oder 9, **dadurch gekennzeichnet**, daß das Heizelement (10) an seinem Umfang mit einem Mantel (13) umgeben ist, der die Oberseite des Heizelementes um eine die Brennstoffprobenmenge definierende Kragenhöhe (14) überragt.

12. Einrichtung nach Anspruch 11, **dadurch gekennzeichnet**, daß der von der Kragenhöhe (14) auf der Heizelement-Oberseite gebildete Raum bündig mit einem Körper (15) vorzugsweise aus porösem Material aufgefüllt ist.

13. Einrichtung nach Anspruch 8 oder 9, **dadurch gekennzeichnet**, daß das Heizelement beidseitig mit einem Körper (15) vorzugsweise aus porösem Material versehen ist.

14. Einrichtung nach Anspruch 12, **dadurch gekennzeichnet**, daß der Körper (15) Ausnehmungen (16) aufweist.

15. Einrichtung nach einem der vorstehenden Ansprüche 8 - 14, **dadurch gekennzeichnet**, daß das Heizelement (10) als Lochplatte ausgeführt ist, wobei die Ausnehmungen (16) der Lochplatte ausgehend von deren Oberseite durchgehend offen oder zur Unterseite hin geschlossen sind.

## Claims

1. Process for utilizing fuels with alcohol additives for a combustion engine which is operated using an electronic mixture-forming system and in which, by means of a fuel sensor for registering the alcohol proportion in a petrol-combustion mixture, a correction signal for changing the mixture composition is generated in a control unit, the fuel sensor consisting of a device for evaporating a fuel sample amount, characterized in that the correction signal is determined in that, in a
- first process step, a fuel sample amount is heated, in that, during the heating, in a
- second process step, the fuel temperature is measured and the temperature gradient of the sample amount of fuel (differential quotient $\frac{dT}{dt}$ of the heating curve $T = f(t)$) is determined continuously, in that, in a

- third process step, with a specified deviation of the determined temperature gradient from a predetermined temperature gradient of a conventional fuel, the specimen amount temperature is measured and an evaporation time measurement is started, a reproducible heating power profile being used as basis, in that, in a
- fourth process step, the sample temperature is measured, on ascertaining a temperature gradient comparable with the starting temperature gradient of the conventional fuel, the evaporation time measurement is stopped and the entire evaporation time is ascertained, in that, in a
- fifth process step, a correction signal is formed from the determined temperature values and evaporation time values by a comparison with comparative values stored in a characteristic data store of the control unit or with alcohol-type-specific characteristic data, in that, in a
- sixth process step, engine control parameters can be changed by means of the correction signal and in that, in a
- seventh process step, the correction signal formed is stored until it is confirmed or updated in a further fuel sample measurement.

2. Process according to the precharacterizing clause of Claim 1, characterized in that the correction signal is determined in that, in a
    - first process step, a fuel sample amount is heated, in that, during the heating, in a
    - second process step, the fuel temperature is measured and the temperature rise is monitored until a first temperature threshold, below the boiling range of an alcohol, is reached, in that, in a
    - third process step, when the first temperature threshold is exceeded, an evaporation time measurement is started, a reproducible heating-power profile being used as basis, in that, in a
    - fourth process step, the temperature rise is monitored until a second temperature threshold, above the boiling range of an alcohol, is reached, the evaporation time measurement is stopped and the entire evaporation time is ascertained, in that, in a
    - fifth process step, a correction signal is formed from the determined evaporation time values by a comparison with comparative values stored in a characteristic data store of the control unit or with alcohol-type-specific characteristic data, in that, in a

- sixth process step, engine control parameters can be changed by means of the correction signal and in that, in a
- seventh process step, the correction signal formed is stored until it is confirmed or updated in a further fuel sample measurement.

3. Process according to Claim 2, characterized in that one or more temperature thresholds are additionally provided the exceeding of which within a specified time span after the first start of the evaporation time measurement, a new evaporation time measurement is started.

4. Process according to Claim 1, 2 or 3, characterized in that the fuel sample amounts is [sic] heated with constant or variable voltage until the start of the evaporation time measurement.

5. Process according to Claim 1, 2, 3 or 4, characterized in that the correction signal stored in the seventh process step is also used for a restart of the combustion engine until it is confirmed or updated according to a further fuel specimen measurement.

6. Process according to Claim 1, 2, 3 or 4, characterized in that, on starting of the combustion engine from a specific filling level of the fuel tank, no correction signal or a correction signal corresponding to that of regular-grade fuel is specified on switching off the combustion engine until the updated correction signal is available.

7. Process according to Claim 5 or 6, characterized in that, with the availability of a lambda signal, an adaptation of the correction signal can take place.

8. Device with means for carrying out the process according to Claims 1-7, the device for evaporating a fuel sample amount consisting of a housing with an evaporation chamber having sensors which can be heated by means of a heating element and having inflow outflow channels, characterized in that the filling of the evaporation chamber (2) is controlled via a valve unit (6) which has a valve closing body (8) which interacts with a spring (7) and opens and closes the inflow outflow channels (3, 4) with the aid of a magnet coil (9).

9. Device according to Claim 8, characterized in that the heating element is a PTC element (10).

10. Device according to Claim 8 or 9, characterized in that, in the evaporation chamber (2), a temperature sensor is arranged in the vicinity of, or in contact with, the heating element (10) of the evaporation chamber (2).

11. Device according to Claim 8 or 9, characterized in that the heating element (10) is surrounded on its periphery by a jacket (13) which projects beyond the top side of the heating element by a protrusion height (14) defining the fuel sample amount.

12. Device according to Claim 11, characterized in that the space formed on the heating-element top side by the protrusion height (14) is filled up flush by a body (15), preferably made of porous material.

13. Device according to Claim 8 or 9, characterized in that the heating element is provided on both sides with a body (15), preferably made of porous material.

14. Device according to Claim 12, characterized in that the body (15) has recesses (16).

15. Device according to one of the preceding Claims 8 - 14, characterized in that the heating element (10) is designed as a perforated plate, the recesses (16) of the perforated plate being open throughout, starting from their top side, or being closed towards the bottom side.

**Revendications**

1. Procédé pour utiliser des combustibles avec additifs d'alcool pour un moteur à combustion interne, exploité avec un système électronique de formation du mélange, dans lequel un signal correctif est produit dans un dispositif de commande pour modifier la composition du mélange, au moyen d'un capteur de carburant pour la détection de la part d'alcool dans un mélange combustible d'essence, le capteur de carburant se composant d'un dispositif pour l'évaporation d'une quantité d'échantillon de combustible, caractérisé en ce que le signal correctif est déterminé par le fait que
   - une quantité d'échantillon de combustible est chauffée dans une première phase du procédé, que, pendant le chauffage,
   - la température du combustible est mesurée dans une deuxième phase du procédé, et le gradient de température de la quantité d'échantillon de combustible (quotient différentiel dT/dt de la courbe de chauffage T = f(t)) est déterminé en continu, que,
   - pour un écart fixé entre le gradient de température déterminé et un gradient de température prédéfini d'un combustible courant, la température de la quantité d'échantillon est mesurée dans une troisième phase du procédé, et une mesure du temps d'évaporation est mise en route, en partant d'une courbe de puissance calorifique reproductible, que,
   - en cas d'établissement d'un gradient de température comparable au gradient de base du combustible courant, la température de l'échantillon est mesurée dans une quatrième phase du procédé, la mesure du temps d'évaporation est stoppée, et le temps d'évaporation total est établi, que,
   - un signal correctif est formé dans une cinquième phase du procédé, à partir des valeurs de température et de temps d'évaporation déterminées, par une comparaison avec des valeurs de référence mémorisées dans une mémoire de caractéristiques du dispositif de commande, ou avec des caractéristiques spécifiques aux types d'alcool, que,
   - des grandeurs de commande du moteur sont modifiables au moyen du signal correctif dans une sixième phase du procédé, et que,
   - le signal correctif formé est mémorisé dans une septième phase du procédé, jusqu'à sa confirmation et/ou son actualisation lors d'une nouvelle mesure d'échantillons de combustible.

2. Procédé suivant le préambule de la revendication 1, caractérisé en ce que le signal correctif est déterminé par le fait que
   - une quantité d'échantillon de combustible est chauffée dans une première phase du procédé, que, pendant le chauffage,
   - la température du combustible est mesurée dans une deuxième phase du procédé et la montée en température est surveillée, jusqu'à ce qu'un premier seuil de température, inférieur au domaine d'ébullition d'un alcool, soit atteint, que,
   - en cas de dépassement par le haut du premier seuil de température, une mesure du temps d'évaporation est mise en route dans une troisième phase du procédé, en partant d'une courbe de puissance calorifique reproductible, que,
   - la montée en température est surveillée dans une quatrième phase du procédé, jusqu'à ce qu'un second seuil de température, supérieur au domaine d'ébullition d'un alcool, soit atteint, la mesure du temps d'évaporation est stoppée, et le temps d'évaporation total est établi, que,

- un signal correctif est formé dans une cinquième phase du procédé, à partir des valeurs du temps d'évaporation déterminées, par une comparaison avec des valeurs de référence mémorisées dans une mémoire de caractéristiques du dispositif de commande, ou avec des caractéristiques spécifiques aux types d'alcool, que,
- des grandeurs de commande du moteur sont modifiables au moyen du signal correctif dans une sixième phase du procédé, et que,
- le signal correctif formé est mémorisé dans une septième phase du procédé, jusqu'à sa confirmation et/ou son actualisation lors d'une nouvelle mesure d'échantillons de combustible.

3. Procédé suivant la revendication 2, caractérisé en ce qu'un ou plusieurs seuils de température supplémentaires sont prévus, une nouvelle mesure du temps d'évaporation étant mise en route après le premier départ de cette mesure de temps, en cas de dépassement par le haut de ces seuils dans le cadre d'un intervalle de temps fixé.

4. Procédé suivant l'une des revendications 1, 2 et 3, caractérisé en ce que les quantités d'échantillons de combustible sont chauffées, sous une tension constante ou variable, jusqu'au départ de la mesure du temps d'évaporation.

5. Procédé suivant l'une des revendications 1, 2, 3 et 4, caractérisé en ce que le signal correctif, mémorisé dans la septième phase du procédé, est également utilisé lors d'un redémarrage du moteur à combustion interne, jusqu'à sa confirmation et/ou son actualisation après une nouvelle mesure d'échantillons de combustible.

6. Procédé suivant l'une des revendications 1, 2, 3 et 4, caractérisé en ce que, lors d'un démarrage du moteur à combustion interne, à partir d'un niveau de remplissage défini du réservoir de combustible à l'arrêt du moteur, aucun signal correctif n'est prédéfini, ou un signal correctif, correspondant au combustible normal, est prédéfini, jusqu'à la production du signal correctif actuel.

7. Procédé suivant l'une des revendications 5 et 6, caractérisé en ce que le signal correctif peut être adapté en présence d'un signal lambda.

8. Dispositif avec des moyens pour la réalisation du procédé suivant les revendications 1 à 7, le dispositif pour l'évaporation d'une quantité d'échantillon de combustible se composant d'un carter, avec une chambre d'évaporation qui peut être chauffée au moyen d'un élément chauffant, est dotée de capteurs et de conduits d'arrivée et d'évacuation, caractérisé en ce que le remplissage de la chambre d'évaporation (2) est réglé par une unité de soupape (6), qui présente un corps de fermeture de soupape (8) concourant avec un ressort (7), le corps précité ouvrant et fermant les conduits d'arrivée et d'évacuation (3, 4) à l'aide d'un solénoïde (9).

9. Dispositif suivant la revendication 8, caractérisé en ce que l'élément chauffant est un élément CTP (10).

10. Dispositif suivant l'une des revendications 8 et 9, caractérisé en ce qu'un capteur de température est disposé dans la chambre d'évaporation (2), au voisinage et/ou au contact de l'élément chauffant (10) de la chambre (2).

11. Dispositif suivant l'une des revendications 8 et 9, caractérisé en ce que l'élément chauffant (10) est enveloppé sur son pourtour par une chemise (13), qui dépasse de la face supérieure de l'élément chauffant d'une hauteur de collerette (14), définissant la quantité d'échantillon de combustible.

12. Dispositif suivant la revendication 11, caractérisé en ce que l'espace, formé par la hauteur de collerette (14) sur la face supérieure de l'élément chauffant, est garni à fleur d'un corps (15), d'un matériau poreux de préférence.

13. Dispositif suivant l'une des revendications 8 et 9, caractérisé en ce que l'élément chauffant est muni de part et d'autre d'un corps (15), d'un matériau poreux de préférence.

14. Dispositif suivant la revendication 12, caractérisé en ce que le corps (15) présente des évidements (16).

15. Dispositif suivant l'une quelconque des revendications précédentes 8 à 14, caractérisé en ce que l'élément chauffant (10) est réalisé sous forme de plaque perforée, les évidements (16) de la plaque perforée étant ouverts en continu à partir de la face supérieure de cette dernière, ou étant fermés en direction de la face inférieure.

# Fig.1

Fig. 2

Fig. 3

Fig. 4

Fig. 5

Fig. 6